# EUROPEAN PATENT SPECIFICATION

(11) **EP 1 669 106 B1**
(45) Date of publication and mention of the grant of the patent: **29.07.2015**
(21) Application number: 05023906.0
(22) Date of filing: 03.11.2005
(51) Int. Cl.: A61Q 5/10, A61K 8/22, A61K 8/44, A61K 8/19, A61Q 5/08

(54) **High level carbonate and/or oxidant hair colouring compositions**
Haarfärbezusammensetzung enthaltend eine grosse Menge an Carbonat und / oder Oxidationsmittel
Composition colorante capillaire contenant une teneur elevée de carbonate et / ou oxidante.

(30) Priority: 02.12.2004 EP 04257512
(43) Date of publication of application: 14.06.2006
(73) Proprietor: The Procter & Gamble Company, Cincinnati, OH 45202 (US)
(72) Inventor: Marsh, Jennifer Mary, Henley-on-Thames Oxfordshire, RG9 3PA (GB)
(74) Representative: Boubel, Thomas

(56) References cited:
- EP-A- 1 454 617
- EP-A- 1 495 751
- WO-A-01/62221
- WO-A-01/76545
- WO-A-02/058659
- JP-A- 11 343 219
- US-A- 3 997 659
- US-A1- 2003 196 280
- US-A1- 2004 078 904

## Description

### FIELD OF THE INVENTION

The present invention relates to compositions for the bleaching and colouration of keratinous fibres.

### BACKGROUND OF THE INVENTION

The permanent alteration of the colour of keratinous fibres, in particular human hair, by the application of hair dyes is well known. In order to provide the consumer with the hair colour and the intensity of colour desired, a very complex chemical process is utilized. Permanent hair dyeing formulations typically comprise oxidative hair dye precursors, which can diffuse into the hair through the cuticle and into the cortex, where they can then react with each other and suitable oxidising agents to form the end dye molecules. Due to the larger size of these resultant molecules they are unable to readily diffuse out of the hair during subsequent washing with water and/or detergents; hence delivering the consumer-desired permanency of colour. This reaction typically takes place in an aggressive environment at approximately pH 10 in the presence of both an alkalizing agent and an oxidizing agent. Moreover, the consumer repeats this process regularly in order to maintain the desired hair colour and shade and the intensity of colour and to ensure continual, even coverage of the hair including coverage of new hair growth.

The manufacturer of such products is also required to work within a large number of constraints. Since these products are being placed in direct contact with the consumers' skin, the potential exists for accidental contact with the eye or for ingestion (for example), which can occur during the dyeing process. Therefore, the formulations must meet rigorous safety requirements and not induce any allergic reactions. In addition, to meeting these requirements, the products must also be optically and olfactory pleasing to the consumer. In particular, the products need to meet certain physical parameters in order to ensure that the product can be easily applied to the hair by the consumer to provide the desired effect, without unintentional staining of the consumers' clothes, skin, particularly along the hair line, or other objects.

The manufacturer is also required to provide the hair colouring consumer with a large range of different resulting colours. Some consumers may just wish to enhance the natural colour of the hair, whilst others may wish to cover grey or completely alter the hair colour to a different natural appearing hair colour or a 'synthetic' appearing hair colour. Consequently, the manufacturer may provide over twenty different formulations, of varying colours and shades, to address the range of consumer specific needs. These formulations have to be individually formulated and are typically complex formulae containing a mixture of different dye compounds. As a result the manufacture of such product ranges can be costly and complex.

However, despite the fact that commercial hair dyeing products have been available for many years, the products still exhibit a number of consumer-related deficiencies.

Typically permanent hair dye products will contain an alkali, typically a source of ammonia. This serves the purpose of swelling the hair, allowing the entry of the dye precursor molecules into the hair and also improves the lightening effect of the oxidising agent, which is typically hydrogen peroxide. However, ammonia is also volatile and its associated odour is extremely unpleasant to the consumers' of such products, particularly as these hair dye products are used in close proximity to the nasal region. Hence, it would be highly desirable to provide an oxidative hair colouring and/or bleaching composition which delivers the consumer required lightening level and colour, but which has reduced or eliminated the detectable ammonia odour.

In fact, another deficiency area in current hair colouring products is the provision of hair colouring products which deliver the required hair colour, especially higher levels of lightening, and effective coverage of grey hair. Indeed, whilst the amount of grey hair to be coloured varies considerably from consumer to consumer, the resultant overall appearance of the coloured hair demanded by the consumer should be nearly identical for the naturally pigmented hair, the grey hair on head and new root growth so as to provide even colour deposition from root to tip. Furthermore it is also important that the initial uniform and even colour coverage is maintained during the post dyeing washing and drying cycle.

Delivering high levels of lightening is particularly important in order to provide the required range of blonde shades and for superior grey coverage that the consumer requires. In addition, another requirement for the consumer is the provision of vibrant shades such as reds, where the vibrancy increases as the underlying colour of the hair is lightened. Such products pose particular difficulties to the manufacturer, as they usually require the use of high levels of oxidising agents such as hydrogen peroxide and high levels of ammonia in order to deliver the required lightening effect. However, in addition to the problems associated with the presence of high levels of ammonia and peroxide in these products, they also affect the condition of the hair and may in some cases induce skin irritation on the scalp. An alternative method to deliver these high levels of lightening is to use so called 'Bleach' products which comprise peroxide, ammonia and persulphate in the absence of dyes. These products deliver the necessary lightening, but suffer from the deficiency that the fibre damage is very high with these products. Yet another deficiency with the products that provide high lightening is that oxidative and preformed dyes are not stable in the presence of oxidative systems. This has the consequence that the consumer has to pre-bleach the hair to the desired lightness level and then in a separate step has to apply a product containing either oxidative or preformed dyes.

Hence, it would also be highly desirable to provide an oxidative hair colouring and/or bleaching composition which delivers the required high lightening and/or colour in one step without unnecessary hair damage.

Another critical performance area for the consumer is the time, that is required to fully develop the required colour. In particular, the application of hair dye products is still a relatively time consuming process and it may take the consumer over an hour to mix, apply, wait for the colour to develop and remove the product; before drying and restyling. The majority of current colourant products take a minimum of at least 25 minutes for the final colour to fully develop, necessitating that the consumer has to sit with the product applied to the hair for this period of time. Since for most consumers the hair dyeing process is a regular part of their beauty routine it would be highly desirable if the time required to dye the hair could be reduced whilst still meeting all of the other requirements of: ease of application, low odour, and especially, of course, the delivery of the required hair colour, particularly for consumers requiring significant changes and/or levels of lift in the resultant colour.

There are a large number of dyes available which are used in hair colouring products. However, consumer demand for the delivery of specific colours is such that there is still a need to improve and increase the colours provided by hair colourant manufacturers, particularly for the yellow colour range. The development of new dyes however is extremely costly and time consuming and thus the number of new dyes available does not increase significantly over time.

Hence, it would be further desirable to provide the consumer with a hair colourant, providing improved lift and lightening, improved colour delivery, uptake and durability, and which provides improved colour and colour variations based upon currently available dyes.

It has now been surprisingly found that hair colouring and /or bleaching systems comprising an oxidizing agent and a source of carbonate ions, wherein said composition comprises at least greater than 4% by weight of said oxidising agent or said source of carbonate ions and an alkalising agent utilised at pH 9.5 and below, free of radical scavenger, provide improved lift and lightening especially for blonde shades, excellent dye deposition and grey hair coverage, and improved colour intensity whereby different colours, shades and chromophores are provided. Moreover, the compositions of the present invention also surprisingly exhibit low odour and reduce the hair fibre damage.

A number of attempts have been described in the literature to address at least some of the above identified improvement areas. For example, the use of carbonate has been described in the following hair colouring art. However none of these references disclose the claimed features of the present invention.

EP 435 012 describes hair-dyeing compositions, which require a short dyeing time, create little damage to hair, and no irritating odour after dyeing comprising a carbonate source, a non odour generating alkali hydrogen peroxide and a buffer solution. Similarly EP 1 106 166 describes hair dye compositions comprising ammonia, carbonate (other than ammonia salt), transition metal salt and chelating agent which do not give off an irritating odour, have low skin irritation and can change the hair colour into a lighter tone in a short time. WO01/28508 describes hair colouring formulations comprising oxidising agents and ammonia carbonate or carbamate which deliver improved bleaching and colouring with reduced odour and hair damage without the need for buffering agents, pH modifiers or hair swelling agents. JP01206825 describes a low pungent hair colouring composition comprising ammonia, ammonium salt and carbonate. US2004/0083557 describes hair colouring compositions comprising an oxidative hair dye precursor, a metal cyanate, an alkalizing agent and an oxidizing agent and preferably a metal bicarbonate salt in order to provide good colour lift and low odour.

WO04/014328 describes one step hair colouring compositions comprising peroxide oxidizing agents, specific oxidizing agents, and at least one water soluble carbonate releasing salts which more effectively deliver colour wherein the composition is applied for a period of from 2 to 60 minutes. US2004/0098814 describes a method of permanently colouring hair whereby the hair is subjected to a number of consecutive short treatments whereby the treatment comprises a dye intermediate in a shampoo or conditioner base, a water soluble carbonate releasing salt and a water soluble ammonium salt. US2004/0098816 also describes a method for the gradual permanent colouring of hair which includes subjecting the hair to a number of treatments having a set time interval between them, wherein the treatment compositions comprise ammonium carbonate in combination with a chelant.

### SUMMARY OF THE INVENTION

The present invention relates to a hair colouring composition as claimed in claim 1.

A further aspect the present invention relates to a method of oxidative colouring or oxdaitive bleaching of hair comprising the steps of applying a composition according to the present invention, leaving said composition on the hair for from 2 to 60 minutes and subsequently rinsing said composition from the hair.

Another aspect of the present invention relates to a method of sequential oxidative hair colouring comprising the steps of at least two sequential oxidative hair colour treatments wherein the time period between each treatment is from 1 day to 60 days, and wherein each treatment comprises the steps of providing a composition of the present invention and then applying said composition to the hair and retaining said composition on the hair for a time period of less than 20 minutes and subsequently rinsing said composition from the hair.

A further aspect of the present invention relates to a hair colouring kit as claimed in claim 6.

### DETAILED DESCRIPTION OF THE INVENTION

Currently marketed hair permanent colourant products typically utilize a combination of an alkaliser system, dye precursors and an oxidant to deliver the desired hair colour to the consumer. The alkaliser is typically ammonia or an alkanolamine, such as monoethanolamine, and the oxidant is typically hydrogen peroxide or a solid form of hydrogen peroxide. The final hair colour which is delivered to the consumer is a combination of the result of the underlying bleaching of the melanin pigment in the hair fibre and the delivery of the coloured dye chromophore moities which are either preformed, that is direct dyes, or are formed by oxidation of the dye precursors within the hair fibre.

The optimal pH for such systems is typically pH 10.0. This high pH is necessary in order to produce a sufficient concentration of the perhydroxy anion (HOO-) to give the desired bleaching of the melanin. It has been found that below pH 9.5 the concentration of this species is less than 0.01% of the added hydrogen peroxide concentration (pKa = 11.6) and the amount of melanin bleaching drops dramatically and is hence insufficient to give the desired final colour.

However, as discussed herein above, compositions having a high pH cause many of the disadvantages noted by consumers for these colourant systems. In particular, the level of the volatile ammonia increases at high pH (above pH 9.5) giving increased unpleasant odour. In addition these compositions are unable to give the high lightening the consumer needs and the virbrant colours that the consumer desires. Furthermore, reactive species including the perhydroxy anion reacts with the hair fibre resulting in significant fibre damage. One consequence of this reactivity is that the hydrophilicity of the hair fibres is significantly increased and this causes an increase in the force required to comb the hair compared with hair that has not been coloured. Moreover the higher forces that are exerted during combing and styling result in increased fibre damage to the hair fibres.

It has now been surprisingly found that hair colouring and bleaching compositions comprising the combination of at least one source of peroxymonocarbonate ions, preferably formed *insitu* from a source of hydrogen peroxide and a carbonate ion source, wherein the composition comprises at least greater than 4% by weight of the peroxide or the carbonate source and at least one source of alkalizing agents, at a pH of 9.5 and below can deliver improvements to the level of lift required whilst also providing the desired hair colour results. Moreover, the compositions also reduce the odour and the damage to the hair fibres.

Whilst not wishing to be bound by theory, it is believed that in the present invention the key species responsible for the bleaching of the melanin, namely the peroxymonocarbonate ion (-OC(O)OOH), decomposes at pH values above 9.5 to form oxygen and the hydrogen carbonate ion. At pH values below 7.5 the hydrogen carbonate ion decomposes to form carbon dioxide and water. At pH values of 9.0 the bleaching of the melanin and the final colour observed is at an optimal level. Thus surprisingly, the present invention allows for the delivery of improved lift, that is hair lightening which is a highly desirable consumer need. Furthermore, compositions having a pH lower than 9.5 have the benefit that the unpleasant ammonia odour is significantly reduced which allows for the formation of a hair colouring product that delivers the desired lightening and colour with a pleasant, cosmetic-like odour. In addition, the peroxymonocarbonate ions at the lower pH of 9.0 causes less fibre damage than current colouring systems. In particular this gives better hair fibre appearance and thus improved hair shine and colour appearance.

Whilst not being bound by theory it is further believed the claimed compositions improve the stability of both oxidative and preformed dyes verses conventional peroxide and ammonium systems or persulphate and peroxide systems whilst still allowing for higher lightening levels.

In addition, whilst not being bound by theory it is believed the claimed composition deliver different colours due to the formation of different ratios of chromophores in the carbonate system verses the ammonium hydroxide/ peroxide system. It is also believed that the carbonate system allows for faster colour formation kinetics resulting in the more rapid delivery of the final colour to the consumer's hair. This means the development time on the consumer's hair can be significantly reduced to less than 25 minutes if required. In addition, the faster dye kinetics means that more dye is taken up by the hair and the wash fade performance is improved.

### Oxidizing agent

The compositions according to the present invention thus comprise a source of peroxymonocarbonate ions. These ions are typically formed insitu from the reaction between a source of hydrogen peroxide and carbonate ion. Consequently, the compositions according to the present invention comprise or are used in combination with a composition that comprises at least one source of an oxidizing agent. Preferred oxidizing agents for use herein are water-soluble peroxygen oxidizing agents. "Water-soluble" as defined herein means that in standard condition at least 0.1 g, preferably 1g, more preferably 10g of said oxidizing agent can be dissolved in 1 liter of deionized water. The oxidizing agents are valuable for the initial solubilisation and decolorisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and/or peroxidases may also be used. Mixtures of two or more such oxidizing agents can be used if desired. The oxidizing agents may be provided in aqueous solution or as a powder which is dissolved prior to use. Preferred for use in the compositions according to the present invention are hydrogen peroxide, percarbonate (which may be used to provide a source of both oxidizing agent and carbonate ions), persulphates and combinations thereof.

According to the present invention the compositions comprise from about 0.1 % to 10% by weight, preferably from 1% to 7% by weight, and most preferably from 2% to 5% by weight of an oxidizing agent.

### Carbonate ion source

According to the present invention the compositions thus also comprise at least a source of carbonate ions or carbamate ions or hydrocarbonate ions or any mixture thereof. Any source of these ions may be utilized. Suitable sources for use herein include sodium, potassium, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate and mixtures thereof.

The compositions of the present invention may comprise from 0.1% to 15%, preferably from 0.1% to 10% by weight, more preferably from 1% to 8% by weight of the carbonate ion. Preferably, if present, the ammonium ions and carbonate ions are present in the composition at a weight ratio of from 3:1 to 1:10, preferably 2:1 to 1:5. In a particularly preferred embodiment of the present invention the ammonium ions and carbonate ion sources are provided by a single source such as ammonium carbonate, ammonium hydrogen carbonate, ammonium hydrocarbonate or mixtures thereof.

According to the present invention the oxidative colouring compositions comprise at least greater than 4%, preferably at least 4.5%, more preferably at least greater than 4.5% to 15%, even more preferably at least 5%, most preferably at least 6% by weight of said source of hydrogen peroxide or said source of carbonate, carbamate and/or hydrogen carbonate.

### Source of alkalizing agent

According to the present invention the composition also comprises at least one source of alkalizing agent, wherein the source of alkalizing agent is a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonia and mixtures thereof.

The compositions of the present invention may comprise from 0.1% to 10% by weight, preferably from 0.5% to 5%, most preferably from 1% to 3% of an alkalizing agent, preferably ammonium ions.

### pH

The compositions of the present invention have a pH of from 7.5 to 9.5, more preferably from 8.4 to 9.5 and most preferably from 8.5 to 9.4 and even more preferably pH 9.0.

Preferably the compositions of the present invention are prepared such that prior to application to the hair fibres the pH of the composition is no greater than pH 9.5. However, in another embodiment of the present invention the compositions may be formulated such that the pH is up to 9.5 during the time period of application of the composition to the hair fibres prior to removal therefrom. Preferably, the pH is up to 9.5 for at least 50% of the time period, preferably at least 70%, most preferably at least 80% of the time period of application of the composition to the hair. More preferably, the pH of the composition is up to pH 9.5 within 10 minutes, preferably within 5 minutes of application to the hair fibres.

The pH of the compositions can be determined by using either a Mettler Toledo MP220 or a MP225 pH equipment, fitted with a standard laboratory pH electrode. The equipment is calibrated before each use using standard calibration buffers and using the standard calibration procedure.

It is known that for good lightening and good colour formation the final formulation should have a good buffering capacity or reserve alkalinity (the ability of the system to resist the pH shift that would otherwise be caused by addition of acid). The reserve alkalinity is measured using a Mettler DL70 auto-titrator with 0.1N methanolic hydrochloric acid being added to 0.7mLof thoroughly mixed colourant product in 50mL of methanol. The electrode is calibrated and then used to measure the amount of acid required to reach the sharpest end point triggered by a rapid change in pH. Using this method it has been determined that a reserve alkalinity of at least 0.2ml of 0.1N of ethanolic hydrochloric acid and preferably above 0.4 is required for good lightening and colouring. Suitable buffering systems include ammonia/ammonium acetate mixtures, monoethanolamine tetrasodium pyrophosphate, isopropanolamine, benzoic acid.

### Radical Scavenger

According to the present invention the compositions are typically free of a source of radical scavenger. The term free as used herein refers to compositions comprising less than 3%, preferable less than 2% more preferably less than 1% even more preferably less than 0.1 % even more preferably less than 0.01% and even more preferably less than 0.001 % and most preferably substantially free of a source of radical scavenger.

As used herein the term radical scavenger refers to compounds according to the general formula: (I): R¹-Y-C(H)(R³)-R⁴-(C(H)(R⁵)-Y-R⁶)ₙ
wherein Y is NR², O, or S, preferably NR², n is 0 to 2, and wherein R⁴ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein R⁴ can be connected to R³ or R⁵ to create a 5, 6 or 7 membered ring; and wherein R¹, R², R³, R⁵, and R⁶ are monovalent and are selected independently from: (a), (b) and (c) described herein above, or H.

Preferably, R⁴ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably R⁴ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.

Preferably, the R⁴ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; and most preferred is O.

Preferably, R¹, R², R³, R⁵, and R⁶ are selected independently from any of the systems defined for R⁴ above, and H.

In alternative embodiments, any of R¹, R², R³, R⁴, R⁵, and R⁶ groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, mono-, poly-, or per-fluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group of N-linked monovalent substituents consisting of NA¹A², (NA¹A²A³)⁺, NC, NA¹OA², NA¹SA², NCO, NCS, NO₂, N=NA¹, N=NOA¹, NA¹CN, NA¹NA²A³; (e) the group of monovalent substituents consisting of COOA¹, CON₃, CONA¹₂, CONA¹COA², C(=NA¹)NA¹A², CHO, CHS, CN, NC, and X; and (f) the group consisting fluoroalkyl monovalent substituents consisting of mono-, poly-, or per-fluoro alkyl systems comprising from 1 to 12 carbon atoms and 0 to 4 heteroatoms.

For the groups (b) to (e), described above, A¹, A², and A³ are monovalent and are independently selected from: (1) H, (2) substituted or unsubstituted, straight or branched, alkyl, mono- or poly-unsaturated alkyl, heteroalkyl, aliphatic, heteroaliphatic, or heteroolefinic systems, (3) substituted or unsubstituted, mono- or poly-cyclic aliphatic, aryl, or heterocyclic systems, or (4) substituted or unsubstituted, mono-, poly-, or per-fluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.

Preferred substituents for use herein include those having a Hammett Sigma Para (σₚ) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375). Without being limited by theory, it is believed that substituents having sigma para values in the chosen ranges, when substituted onto R¹ and/or R², may improve the compound's toxicological profile without unduly adding an unfavourable increase in molecular weight that may interfere with the molecule's ability to penetrate the hair shaft. Some preferred substituents and their Hammett Sigma Para values are shown below, in Table A. Additional substituents and their values are shown in March, at page 370.

**Table A**

| Substituen t | NH₂ | OH | H | COO- | Cl | COOH | CF₃ |
|---|---|---|---|---|---|---|---|
| □ₚ | -0.57 | -0.38 | 0 | 0.11 | 0.24 | 0.44 | 0.53 |

Preferably the above defined radical scavengers have a pKa of more than 7 to prevent the protonation of the nitrogen.

Preferably the present invention further does not comprise radical scavengers according to the general formula (II) : 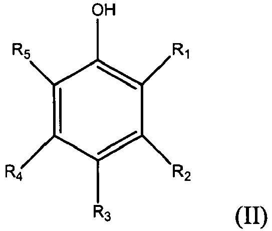 wherein R₁, R₂, R₃, R₄, and R₅ are each independently selected from H, COO⁻M⁺, Cl, Br, SO₃⁻M⁺, NO₂, OCH₃, OH or a C¹ to C¹⁰ primary or secondary alkyl and M is either H or alkali metal. Preferably, the above-described radical scavengers have a pKa of more than 8.5 to ensure protonation of the hydroxy goup.

Preferably the present invention further also does not comprise radical scavengers according to those selected from group (III) benzylamine, imidazole, di-tert-butylhydroxytoluene, hydroquinone, guanine, pyrazine, piperidine, morpholine, methylmorpholine, 2methyoxyethylamine, and mixtures thereof.

Even more preferably the present invention does not comprise radical scavenger defined as a species that can react with a carbonate radical to convert the carbonate radical by a series of fast reactions to a less reactive species, i.e. a carbonate radical scavenger.

Whilst not being bound by theory, it is believed that the ability of the radical scavenger to convert the carbonate radical (as described hereinabove) is dependant upon the energy of the charge transfer reaction as shown below: (The calculation of the energy of the charge transfer reaction is detailed hereinafter.)

Scavenger + CO₃^{*-} → Scavenger^{*+} + CO₃²⁻

wherein the energy of the reaction is defined by:-
ΔHᵣ =ΔHᵣ (products) - ΔH_{f} (reactants)
   = ΔH_{f} (Scavenger^{*+}) + ΔH_{f} (CO₃²⁻ )- ΔH_{f} (Scavenger) - ΔH_{f} (CO₃^{*-})
According to the present invention the composition do not comprise a radical scavenger having an energy of reaction of from 0 kcal/mol to 14 kcal/mol, preferably from 1.5 kcal/mol to 9 kcal/mol.

### Additional components

The compositions of the present invention may further comprise additional ingredients which include, but are not limited to, hair dyeing agents such as oxidative dye precursors, non-oxidative dyes, thickeners, solvents, enzymes, surfactants, conditioning agents, carriers, antioxidants, stabilizers, chelants, perming actives, perfume, reducing agents (thiolactic acid), hair swelling agents and/or polymers. Some of these additional components are detailed hereafter.

### Hair dyes

The hair colouring compositions of the present invention further comprise hair dye materials. Such compositions comprise oxidative hair dye precursors (also known as primary intermediates) that will deliver a variety of hair colors to the hair. These small molecules are activated by the oxidizing agent and react with further molecules to form a larger colored complex in the hair shaft. The precursors can be used alone or in combination with other precursors, and one or more can be used in combination with one or more couplers. Couplers (also known as color modifiers or secondary intermediates) are generally colorless molecules that can form colors in the presence of activated precursors, and are used with other precursors or couplers to generate specific color effects or to stabilize the color.

The choice of precursors and couplers will be determined by the color, shade and intensity of coloration that is desired. The precursors and couplers can be used herein, singly or in combination, to provide dyes having a variety of shades ranging from ash blonde to black. These compounds are well known in the art, and include aromatic diamines, aminophenols, and their derivatives (a representative but not exhaustive list of oxidation dye precursor can be found in Sagarin, "Cosmetic Science and Technology", "Interscience, Special Edn. Vol. 2 pages 308 to 310). It is to be understood that the precursors detailed below are only by way of example and are not intended to limit the compositions and processes herein. These are:
1,7-Dihydroxynaphthalene (1,7-NAPHTHALENEDIOL), 1,3-Diaminobenzene (m-PHENYLENEDIAMINE), 1-Methyl-2,5-diaminobenzene (TOLUENE-2,5-DIAMINE), 1,4-Diaminobenzene (p-PHENYLENEDIAMINE), 1,3-Dihydroxybenzene (RESORCINOL), 1,3-Dihydroxy-4-chlorobenzene, (4-CHLORORESORCINOL), 1-Hydroxy-2-aminobenzene, (o-AMINOPHENOL), 1-Hydroxy-3-aminobenzene (m-AMINOPHENOL), 1-Hydroxy-4-amino-benzene (p-AMINOPHENOL), 1-Hydroxynaphthalene (1-NAPHTHOL), 1,5-Dihydroxynaphthalene (1,5-NAPHTHALENEDIOL), 2,7-dihydroxynaphthalene (2,7-NAPHTHELENEDIOL) 1-Hydroxy-2,4-diaminobenzene (4-DIAMINOPHENOL), 1,4-Dihydroxybenzene (HYDROQUINONE), 1-Hydroxy-4-methylaminobenzene (p-METHYLAMINOPHENOL), 6-Hydroxybenzo-morpholine (HYDROXYBENZOMORPHOLINE), 1-Methyl-2-hydroxy-4-aminobenzene (4-AMINO-2-HYDROXY-TOLUENE), 3,4-Diaminobenzoic acid (3,4-DIAMINOBENZOIC ACID), 1-Methyl-2-hydroxy-4-(2'-hydroxyethyl)aminobenzene (2-METHYL-5-HYDROXY-ETHYLAMINO-PHENOL), 1,2,4-Trihydroxybenzene (1,2,4-TRIHYDROXYBENZENE), 1-Phenol-3-methylpyrazol-5-on (PHENYLMETHYLPYRAZOLONE), 1-(2'-Hydroxyethyloxy)-2,4-diaminobenzene (2,4-DIAMINOPHENOXY-ETHANOL HCL), 1-Hydroxy-3-amino-2,4-dichlorobenzene (3-AMINO-2,4-DICHLORO-PHENOL), 1,3-Dihydroxy-2-methylbenzene (2-METHYLRESORCINOL), 1-Amino-4-bis-(2'-hydroxyethyl)aminobenzene (N,N-BIS(2-HYDROXY-ETHYL)-p-PHENYLENE-DIAMINE), 2,4,5,6-Tetraaminopyrimidine (HC Red 16), 1-Hydroxy-3-methyl-4-aminobenzene (4-AMINO-m-CRESOL), 1-Hydroxy-2-amino-5-methylbenzene (6-AMINO-m-CRESOL), 1,3-Bis-(2,4-Diaminophenoxy)propane (1,3-BIS-(2,4-DIAMINO-PHENOXY)-PROPANE),1-(2'-Hydroxyethyl)-2,5-diaminobenzene (HYDROXYETHYL-p-PHENYLENE DIAMINE SULPHATE), 1-Methoxy-2-amino-4-(2'-hydroxyethylamino)benzene, (2-AMINO-4-HYDROXYETHYLAMINOANISOLE) 1-Hydroxy-2-methyl-5-amino-6-chlorobenzene (5-AMINO-6-CHLORO-o-CRESOL), 1-Hydroxy-2-amino-6-methylbenzene (6-AMINO-o-CRESOL), 1-(2'-Hydroxyethyl)-amino-3,4-methylenedioxybenzene (HYDROXYETHYL-3,4-METHYLENEDIOXY-ANILINE HCl), 2,6-Dihydroxy-3,4-dimethylpyridine (2,6-DIHYDROXY-3,4-DIMETHYLPYRIDINE), 3,5-Diamino-2,6-dimethoxypyridine (2,6-DIMETHOXY-3,5-PYRIDINEDIAMINE), 5,6-Dihydroxyindole (,DIHYDROXY-INDOLE), 4-Amino-2-aminomethylphenol (2-AMINOETHYL-p-AMINO-PHENOL HCl), 2,4-Diamino-5-methylphenetol (2,4-DIAMINO-5-METHYL-PHENETOLE HCl), 2,4-Diamino-5-(2'-hydroxyethyloxy)toluene (2,4-DIAMINO-5-METHYLPHENOXYETHANOL HCl), 5-Amino-4-chloro-2-methylphenol (5-AMINO-4-CHLORO-o-CRESOL), 4-Amino-1-hydroxy-2-(2'-hydroxyethylaminomethyl)benzene HYDROXYETHYLAMINOMETHYL-p-AMINO PHENOL HCl), 4-Amino-1-hydroxy-2-methoxymethylbenzene (2-METHOXYMETHYL-p-AMINOPHENOL HCl), 1,3-Bis(N(2-Hydroxyethyl)N(4-amino-phenyl)amino)-2-propanol (HYDROXYPROPYL-BIS-(N-HYDROXY-ETHYL-p-PHENYLENEDIAMINE)HCL), 6-Hydorxyindole (6-HYDROXY-INDOLE), 2,3-Indolinedione (ISATIN), 3-Amino-2-methylamino-6-methoxypyridine (HC BLUE NO. 7), 1-Phenyl-3-methyl-5-pyrazolone-2,4-dihydro-5,2-phenyl-3H-pyrazole-3 -one, 2-Amino-3-hydroxypyridine (2-AMINO-3-HYDROXYPYRIDINE), 5-Amino-salicylic acid, 1-Methyl-2,6-bis(2-hydroxy-ethylamino)benzene (2,6-HYDROXYETHYLAMINO-TOLUENE), 4-Hydroxy-2,5,6-triaminopyrimidine (2,5,6-TRIAMINO-4-PYRIMIDINOL SULPHATE), 2,2'-[1,2-Ethanediyl-bis-(oxy-2,1-ethanediyloxy)]-bis-benzene-1,4-diamine (PEG-3,2',2'-DI-p -PHENYLENEDIAMINE), 5,6-Dihydroxyindoline (DIHYDROXYINDOLINE), N,N-Dimethyl-3-ureidoaniline (m-DIMETHYL-AMINO-PHENYLUREA), 2,4-Diamino-5-fluortoluenesulfatehydrate (4-FLUORO-6-METHYL-m-PHENYLENEDIAMINE SULPHATE) and 1-Acetoxy-2-methylnaphthalene (1-HYDROXYYETHYL-4,5-DIAMINOPYRAZOLE SULPHATE). These can be used in the molecular form or in the form of peroxide-compatible salts.

The hair colouring compositions of the present invention may also include non oxidative hair dyes i.e. direct dyes which may be used alone or in combination with the above described oxidative dyes. Suitable direct dyes include azo or anthraquinone dyes and nitro derivatives of the benzene series and mixtures thereof. Such direct dyes are particularly useful to deliver shade modification or highlights. Particularly preferred are Basic Red 51, Basic Orange 31, Basic Yellow 87 and mixtures thereof.

The hair dye compositions of the present invention will generally comprise from 0.001% to 10% of dyes. For example compositions providing low intensity dyeing such as natural blonde to light brown hair shades generally comprise from 0.001% to 5%, preferably from 0.1% to 2%, more preferably from 0.2% to 1% by weight of dyeing composition of precursors and couplers. Darker shades such as browns and black typically comprise from 0.001% to 10% by weight, preferably from 0.05% to 7% by weight, more preferably form 1% to 5% of precursors and couplers.

In addition, the compositions of the present invention may further comprise from 0.001 to 1.0% by weight of a pigment or lake to provide a visual indication of where the composition was applied to the hair. Suitable pigments include, for example, ultramarine blue, D&C yellow No. 10 aluminium lake and mixtures thereof.

### Surfactants

The compositions according to the present invention may further comprise at least 0.01 % of a surfactant. Surfactants suitable for use herein generally have a lipophilic chain length of from 8 to 30 carbon atoms and can be selected from anionic, nonionic, amphoteric and cationic surfactants and mixtures thereof.

### Polymers

The composition of the present invention may optionally further comprise at least 0.01 % of polymer. Whilst not intending to be limiting, the polymer can be chosen, for example, from associative polymers, crosslinked acrylic acid homopolymers, crosslinked copolymers of (meth)acrylic acid and of (C1-C6)alkyl acrylate or polysaccharides. The polymer may serve as a thickening agent and also serve as conditioning agents, as described below. The polymer will generally be used at levels of from 0.01% to 20.0% by weight of the composition, preferably of from 0.1% to 5%.

### Conditioning agent

The compositions of the present invention may comprise or are used in combination with a composition comprising a conditioning agent. Conditioning agents suitable for use herein are selected from silicone materials, amino silicones, fatty alcohols, polymeric resins, polyol carboxylic acid esters, cationic polymers, cationic surfactants, insoluble oils and oil derived materials and mixtures thereof. Additional materials include mineral oils and other oils such as glycerin and sorbitol. Particularly useful conditioning materials are cationic polymers and silicones.

Conditioners of the cationic polymer type can be chosen from those comprising units of at least one amino groups chosen from primary, secondary, tertiary and quaternary amine groups that may either form part of the main polymer chain, or be borne by a side substituent that is directly attached to the main polymer chain.

Silicones can be selected from polyalkylsilioxane oils, linear polydiemthylsiloxane oils containing trimethylsilyl or hydroxydimethylsiloxane endgroups, polymethylphenylsiloxane polydimethylphenylsiloxane or polydimethyldiphenylsiloxane oils, silicone resins, organofunctional siloxanes having in their general structure one or a number of organofunctional group(s), the same or different, attached directly to the siloxane chain or mixtures thereof. Said organofunctional group(s) are selected from: polyethyleneoxy and / or polypropyleneoxy groups, (per)fluorinated groups, thiol groups, substituted or unsubstituted amino groups, carboxylate groups, hydroxylated groups, alkoxylated groups, quatemium ammonium groups, amphoteric and betaine groups. The silicone can either be used as a neat fluid or in the form of an pre-formed emulsion.

The conditioning agent will generally be used at levels of from 0.05% to 20% by weight of the composition, preferably of from 0.1% to 15%, more preferably of from 0.2% to 10%, even more preferably of from 0.2% to 2%.

### Chelants

According to the present invention the compositions may comprise chelants. Chelants are well known in the art and refer to a molecule or a mixture of different molecules each capable of forming a chelate with a metal ion. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996) both incorporated herein by reference.

Examples of chelants suitable for use herein include EDDS (ethylenediaminedisuccinic acid), carboxylic acids (in particular aminocarboxylic acids), phosphonic acids (in particular aminophosphonic acids) and polyphosphoric acids (in particular linear polyphosphoric acids), their salts and derivatives.

Chelants may be incorporated into the composition of the present invention as stabilizers and or preservatives. In addition, it has also been found that chelants provide hair fibre damage benefits and thus they may be utilized in order to further improve the hair damage profile of the present invention. Levels of chelants in the present invention may be as low as 0.1%, preferably at least 0.25%, more preferably 0.5% for the most effective chelants such as diamine-N,N'-dipolyacid and monoamine monoamide-N,N'-dipolyacid chelants (for example EDDS). Less effective chelants will be more preferably used at levels of at least 1%, even more preferably above 2% by weight of the composition, depending of the efficiency of the chelant. Levels as high as 10% can be used, but above this level significant formulation issues may arise.

### Solvents

Suitable solvents for use in the compositions of the present invention include, but are not limited to, water, butoxydiglycol, propylene glycol, alcohol (denat.), ethoxydiglycol, isopropylalcohol, hexylene glycol, benzyl alcohol and dipropylene glycol.

Finally, the compositions according to the present invention can be provided in any usual form, such as for example an aqueous composition, a powder, a gel or an oil-in-water emulsion. A preferred form for the compositions according to the present invention is a thickened solution comprising a salt-tolerant thickener or a oil-in-water emulsion.

### Method of Use

It is understood that the examples of methods of use and embodiments described herein are for illustrative purposes only and that various modifications or changes in light thereof will be suggested to one skilled in the art without departing from the scope of the present invention.

Oxidative hair dye compositions are usually sold in kits comprising, in individually packaged components such as separate containers, a dye component (also called "dye cream" for emulsions or "dye liquid" for solutions) comprising the oxidative dye, precursors and alkalizing agent in a suitable carrier, and; a hydrogen peroxide component (also called "hydrogen peroxide cream" for emulsions or "hydrogen peroxide liquid" for solutions) comprising the oxidizing agent. The consumer mixes the dye component and hydrogen peroxide component together immediately before use and applies it onto the hair. The exemplified formulations given in the tables hereinafter illustrate these resulting mixtures.

After working the mixture for a few minutes (to insure uniform application to all of the hair), the oxidative dye composition is allowed to remain on the hair for an amount sufficient for the dyeing to take place (usually 2 to 60 minutes preferably from 30 to 45 minutes). The consumer then rinses his/her hair thoroughly with water and allows it to dry. It is observed that the hair has changed from its original color to the desired color.

When present in the oxidative dye compositions kits, the optional conditioning agent can be provided in a third container. All three compositions can be mixed immediately before use and applied together, or the content of the third container can be applied (after an optional rinse step) as a post-treatment immediately after the oxidative dye composition resulting from the mixture of the other containers.

According to one method of oxidatively colouring hair according to the present invention, this comprises the steps of applying an oxidising hair colouring composition of the present invention having a pH of up to 9.5 when applied to the hair of the consumer, or have a pH that is up to 9.5 for at least 50% of the time period the composition is applied to the hair. Alternatively, the individual compositions may have varying pH levels such that on mixing or application to the consumer the pH is up to 9.5.

According to the present invention the methods of colouring hair also comprise embodiments whereby the composition of the present invention is applied to the hair and preferably the mixture is worked for a few minutes (to insure uniform application to all of the hair). The composition is then allowed to remain on the hair in order for the colour to develop for a time period of less than 20 minutes, preferably less than 15 minutes, more preferably from 5 minutes to 10 minutes, most preferably for 10 minutes. The consumer then rinses his/her hair thoroughly with water and allows it to dry and or styles the hair as usual.

According to a further alternative embodiment of the present invention, the method of colouring the hair is a sequential oxidative hair colouring or method comprising the steps of at least two sequential oxidative hair colour treatments, wherein the time period between each treatment is from 1 to 60 days, preferably from 1 to 40 days, more preferably from 1 to 28 days, even more preferably from 1 to 14 days and most preferably from 1 to 7 days. In such embodiments, the time that the composition is retained on head may be less than 20 minutes and is preferably less than 10 minutes and is most preferably from 2 minutes to 5 minutes.

According to yet a further embodiment of the present invention, the method of colouring hair also comprises embodiments whereby the composition of the present invention is applied to the hair and preferably the mixture is worked for a few minutes (to insure uniform application to all of the hair). The composition is then allowed to remain on the hair in order for the colour to develop for a time period of 10 to 45 minutes. The consumer then rinses his/her hair thoroughly with tap water before the application of either a second application of composition of the present invention or an application of a bleaching system to selected strands. The effect of the two step treatment is to give the consumer a highlighted or streaked effect to her hair. Alternatively the application of the bleaching system can be before the application of the colouring system.

The kits described hereinabove are well known in the art and the composition in each container can be manufactured utilizing any one of the standard approaches, these include a) 'Oil in water' process, b) 'Phase Inversion' process and c) 'One-pot' process.

For example, in a 'One-pot' process, the polymers and chelants would be predissolved in water, the fatty materials added and then the whole composition heated to about 70-80°C. A controlled cooling and optional shearing process to form the final structured product in the case of an emulsion would then follow. Addition of the materials providing source of peroxymonocarbonate ions, dyes and ammonia, and optionally solvents, and pH trimming complete the making process of the dye cream.

In the case of a liquid solution comprising acrylate polymers, these would be formulated into the hydrogen peroxide component. The glycol solvents and fatty components are formulated into the dye component. A structured product is formed when the dye and hydrogen peroxide components are mixed together prior to use of the composition, resulting from deprotonation of the polymer acrylic acid groups as the pH rises, yielding a polymeric micro-gel. Further details on the manufacture of these two-part aqueous composition for coloring hair, which forms a gel on mixing of the two parts can be found in US 5,376,146, Casperson et al. and US 5,393,305, Cohen et al.

The composition of the present invention can also be formulated as 2-part aqueous compositions comprising polyetherpolyurethane as thickening agent (such as Aculyn ® 46) as described in US6,156,076, Casperson et al. and US6,106,578, Jones.

The present invention may be utilized in a variety of packaging and dispensing devices. These dispensing devices can come in the form of separate devices which may be used independently or in combination with one another. Typically, the hair colouring or bleaching compositions are contained within separate single or multi compartment containers so that the compositions can be stored separately from one another before use. The compositions are then mixed together by a mixing means and then applied to the consumer's hair by an application means.

The most common packaging device which can be used for the present invention involves storing the developer in a container such as a bottle, tube, aerosol, or a sachet and separately storing the dye lotion in an additional compartment within the developer container or in a separate container which may be identical such as a dual sachet or aesrosol systems for example or different such as a bottle and tube system.

The consumer may mix the developer lotion and the dye lotion by any means. This may simply involve the use of a mixing bowl into which the lotions are dispensed and then mixed, preferably using a mixing means such as a tool. Alternatively it may involve the addition of one of the lotions into the container of the other lotion, (typically the dye lotion is added to the developer lotion), followed by manual shaking or mixing with a tool. Another system involves the perforation or displacement of a seal located between the separate compartments of the dye and developer lotion within a single container or sachet followed by manual mixing within the container or in a separate and or additional container.

An example of such devices are the so called 'twist and go' devices. These devices allow the consumer to twist the base of a container holding the dye which enables a communication port to open that exposes the base of the bottle holding the dye and the top of the bottle holding the developer. The two components are mixed and the consumer dispenses the product by squeezing the flexible top portion of the bottle for dispensing.

Alternatively more complex devices may be utilised, whereby the lotions are mixed upon actuation of dispensing. An example of such as a complex system is a dual aerosol system e.g. bag-in-can or piston. The dye and developer are stored separately in two aerosol cans within one device, a propellant being used to pressurize the contents of the can or bag in can or piston and a valve providing the control of dispensation. When the consumer actuates the valve, the dye and developer are dispensed simultaneously out of the cans and are mixed together via a static mixer just before dispensing the product onto the hair. The ratio of the dye and developer can be manipulated by the viscosity of the products, the can pressure, or by altering the flow channel sizes through the valve. Additionally, the product can be foamed and delivered via a mousse form.

Another example of such a complex system utilises a dual piston screw system. The dye and the developer are kept in separate piston cylinder systems within the system and when the consumer actuates a button, two screws are rotated such that the dual pistons inside pressurize the liquid in the cylinders and thus force the products to move through a mixing station and out of the nozzle for dispensing. The ratios of the dye and the developer can be manipulated by the diameter of the cylinder of the package. Additionally, an in line static mixer can be used to aid mixing and such a system can be completely disposable or completely refillable.

Yet another system utilises one or more manually actuated pumps. The product may be premixed in a collapsible sachet. When the consumer actuates the pump, the liquid inside the pump is dispensed. As the manually actuated pump returns to the upright position it forces product from a collapsible sachet. Alternatively, a dual system can be installed whereby two sachets and two pumps are used to deliver the dye and the developer lotions to the hair. Alternatively, a single pump connected to two sachets can deliver the product by incorporating the mixing point within the pump. Another embodiment uses a rigid bottle and a dip tube to connect the product to the pump system. Finally, a delaminating bottle can be used in combination with a manually actuated pump where the inner layer of the bottle separates from the outer layer of the bottle which forces the contents of the bottle to be emptied.

Typically these complex systems offer the advantage of product application independently of the orientation of the product.

The devices described herein above can also be used in combination with a product delivery and or application tool to aid application of the product onto the hair. Again these devices may be of a very simple nature such as a nozzle attached to one of the containers or a separate applicator device such as a comb or brush. Such combs and brushes can be adapted in order to achieve particular effects, whether it be quick and even coverage or root/hairline touch up, or highlights or streaks. Alternatively, the container or one of the containers may be provided with a comb attached to or instead of the dispensing nozzle whereby the product is dispensed through hollow tines and dispensing apertures located in the comb tines. The comb tines may be provided with single or multiple openings along the tines to improve product application and evenness especially root to tip. Product dispensation can be achieved by mechanical pressure applied to the container for example delaminating bottles or any of the mechanisms described hereinabove. The comb may be provided on the container such as to facilitate easy application and may be positioned vertically (so called verticomb) or at an angle to allow the consumer to access all areas. All devices may be designed to have inter-changeability, so that a range of different tools for hair application can be provided to the consumer.

The application devices may also include devices which assist in achieving particular effects such as highlighting such as highlighting combs, brushes and tools, foils and highlighting caps.

Additional device technology can be used to assist in the penetration of the product into the hair. Examples of such technology include heating devices, ultraviolet light devices and ultrasound devices.

### Examples

The following examples illustrate oxidative colouring compositions according to the present invention and methods of manufacture thereof. It is understood that the examples and embodiments described herein are for illustrative purposes only and that various modifications or changes in light thereof will be suggested to one skilled in the art without departing from the scope of the present invention.

**Examples of emulsion formulations 1 -1 0**

| | **Formulation** | | | | | | | | | |
|---|---|---|---|---|---|---|---|---|---|---|
| **Ingredient** | **1** | **2** | **3** | **4** | **5** | **6** | **7** | **8** | **9** | **10** |
| Sodium sulphite | 0.1 | 0.1 | 0.1 | 0.1 | 0.1 | 0.1 | 0.1 | 0.1 | 0.1 | 0.1 |
| Ascorbic Acid | 0.1 | 0.1 | 0.1 | 0.1 | 0.1 | 0.1 | 0.1 | 0.1 | 0.1 | 0.1 |
| Ammonium Carbonate | 5 | 6.0 | 5.0 | - | 4.0 | 8.0 | 2.0 | - | 5.0 | 6.0 |
| Potassium Hydrogen Carbonate | - | - | 1.5 | 2.0 | - | - | 2.0 | 8.0 | - | - |
| Ammonium Acetate | - | - | - | 2.0 | - | - | - | 3.0 | - | - |
| Ceteareth-25 | 1.0 | - | - | - | - | - | - | 1.0 | 1.0 | 1.0 |
| Steareth-100 | - | 1.0 | 1.0 | - | - | - | - | - | - | - |
| Sodium Palmytoyl Sarcosinate | - | - | - | - | 1.0 | - | - | - | - | - |
| Sodium Carboxymethyl Lauryl Glucoside | - | - | - | 1.0 | - | - | - | - | - | - |
| Sodium Lauryl Sulfate | - | - | - | - | - | 1.0 | - | - | - | - |
| Behentrimonium Chloride | - | - | - | - | - | - | 1.0 | - | - | - |
| Cetyl Alcohol | 1.6 | - | 2 | 1.5 | 1.5 | 1.5 | 2.5 | 2.5 | 1.6 | 1.6 |
| Stearyl Alcohol | 3.3 | - | 2 | 1.5 | 1.5 | 1.5 | 2.5 | 2.5 | 3.3 | 3.3 |
| Steareth-2 | - | 5 | 1 | - | - | - | - | - | - | - |
| Sodium Benzoate | 0.1 | 0.1 | 0.1 | 0.1 | 0.1 | 0.1 | 0.1 | 0.1 | 0.1 | 0.1 |
| Phenoxyethanol | 0.1 | 0.1 | 0.1 | 0.1 | 0.1 | 0.1 | 0.1 | 0.1 | 0.1 | 0.1 |
| EDTA (tetrasodium salt) | 0.1 | 0.1 | 0.1 | 0.1 | 0.1 | 0.1 | 0.1 | 0.1 | 0.1 | 0.1 |
| p-phenylene diamine | 0.8 | | 0.6 | 0.1 | 0.8 | | 0.6 | 0.1 | 0.8 | |
| p-amino phenol | | 0.3 | | 0.4 | | 0.3 | | 0.4 | | 0.3 |
| 2,5-diaminotoluene sulphate | | 0.1 | 0.2 | | | 0.1 | 0.2 | | | 0.1 |
| m-aminophenol | 0.2 | | 0.1 | | 0.2 | | 0.1 | | 0.2 | |
| Resorcinol | | 0.5 | | 0.4 | | 0.5 | | 0.4 | | 0.5 |
| napthol | 0.03 | | 0.2 | | 0.03 | | 0.2 | | 0.03 | |
| 4-amino-2-hydroxy toluene | | 0.2 | | 0.3 | | 0.2 | | 0.3 | | 0.2 |
| Phenyl methyl pyrazalone | 0.2 | - | - | - | - | 0.1 | - | - | - | - |
| 1-hydroxyethyl-4,5-diamino pyrazole sulphate | 0.3 | - | - | - | - | 0.2 | - | - | - | - |
| Basic red 51 | - | 0.1 | - | - | - | - | 0.2 | - | - | - |
| Basic yellow 87 | - | 0.2 | - | - | - | - | 0.3 | - | - | - |
| Hydrogen Peroxide (35% active) | 8.6 | 8.6 | 8.6 | 12.9 | 17 | 17 | 17 | 10.7 | 10.7 | 10.7 |
| Amodimethicone (Belsil ADM1100) | 1.5 | - | - | - | - | - | - | - | 1.0 | - |
| Trimethylsilylamo-dimethicone (SF1708 ) | - | 0.5 | - | - | - | - | - | 2.0 | - | - |
| Polyquaternium-22 (Merquat 295) | - | - | 2.0 | - | 0.1 | - | - | - | - | - |
| Polyquaternium-37 & Mineral oil (Salcare SC95) | - | - | - | 0.5 | 0.1 | - | - | - | - | - |
| Polyquaternium 10 (Polymer JR30M) | - | - | - | - | - | 0.2 | 0.2 | - | - | - |
| Dicetyldimonium Chloride | - | - | - | - | - | - | - | - | 0.2 | - |
| Xanthan gum | 0.1 | 0.5 | - | - | 0.2 | - | - | - | - | - |
| Cetyl hydroxyethyl Cellulose (Natrosol 330CS Plus) | - | - | 0.8 | - | - | - | - | - | - | - |
| pH adjust to pH 9.0 | qs | qs | qs | qs | qs | qs | qs | qs | qs | qs |
| Water | qs | qs | qs | qs | qs | qs | qs | qs | qs | qs |

**Examples of emulsion formulations 11 - 20**

| | **Formulation** | | | | | | | | | |
|---|---|---|---|---|---|---|---|---|---|---|
| **Ingredient** | **11** | **12** | **13** | **14** | **15** | **16** | **17** | **18** | **19** | **20** |
| Sodium sulphite | 0.1 | 0.1 | 0.1 | 0.1 | 0.1 | 0.1 | 0.1 | 0.1 | 0.1 | 0.1 |
| Ascorbic Acid | 0.1 | 0.1 | 0.1 | 0.1 | 0.1 | 0.1 | 0.1 | 0.1 | 0.1 | 0.1 |
| Ammonium Carbonate | 5.0 | 6.0 | 5.0 | - | 4.0 | 8.0 | 2.0 | - | 5.0 | 6.0 |
| Potassium Hydrogen Carbonate | - | - | 1.5 | 2.0 | - | - | 2.0 | 8.0 | - | - |
| Ammonium Acetate | - | - | - | 2.0 | - | - | - | 3.0 | - | - |
| Crodafos® CES | 2.0 | 3.0 | 1.5 | 3.0 | 3.0 | 3.0 | 3.0 | 3.0 | 3.0 | 3.0 |
| EDTA (tetrasodium salt) | 0.1 | 0.1 | 0.1 | 0.1 | 0.1 | 0.1 | 0.1 | 0.1 | 0.1 | 0.1 |
| p-phenylene diamine | 0.8 | | 0.6 | 0.1 | 0.8 | | 0.6 | 0.1 | 0.8 | |
| p-amino phenol | | 0.3 | | 0.4 | | 0.3 | | 0.4 | | 0.3 |
| 2,5-diaminotoluene sulphate | | 0.1 | 0.2 | | | 0.1 | 0.2 | | | 0.1 |
| m-aminophenol | 0.2 | | 0.1 | | 0.2 | | 0.1 | | 0.2 | |
| Resorcinol | | 0.5 | | 0.4 | | 0.5 | | 0.4 | | 0.5 |
| napthol | 0.03 | | 0.2 | | 0.03 | | 0.2 | | 0.03 | |
| 4-amino-2-hydroxy toluene | | 0.2 | | 0.3 | | 0.2 | | 0.3 | | 0.2 |
| Phenyl methyl pyrazalone | 0.2 | - | - | - | - | 0.1 | - | - | - | - |
| 1-hydroxyethyl-4,5-diamino pyrazole sulphate | 0.3 | - | - | - | - | 0.2 | - | - | - | - |
| Basic red 51 | - | 0.1 | - | - | - | - | 0.2 | - | - | - |
| Basic yellow 87 | - | 0.2 | - | - | - | - | 0.3 | - | - | - |
| Hydrogen Peroxide (35% active) | 8.6 | 8.6 | 8.6 | 12.9 | 17 | 17 | 17 | 10.7 | 10.7 | 10.7 |
| Amodimethicone (Belsil ADM1100) | 1.5 | - | - | - | - | - | - | - | 1.0 | - |
| Trimethylsilylamo-dimethicone (SF1708 ) | - | - | - | - | - | - | - | 2.0 | - | - |
| Polyquaternium-22 (Merquat 295) | - | - | 2.0 | - | 0.5 | - | - | - | - | - |
| Polyquaternium-37 & Mineral oil (Salcare SC95) | - | - | - | 0.5 | 0.1 | - | - | - | - | - |
| Polyquaternium 10 (Polymer JR30M) | - | - | - | - | - | 0.2 | 0.2 | - | - | - |
| Dicetyldimonium Chloride | - | - | - | - | - | - | - | - | 0.2 | - |
| Xanthan gum | 0.1 | - | 0.2 | 0.2 | - | - | - | - | - | - |
| Succinoglycan | - | - | - | - | 0.2 | 0.5 | - | - | - | - |
| Carbomer | - | - | - | - | - | - | 1.0 | 0.5 | - | - |
| Acrylates/C 10-30 Alkyl Acrylate Crosspolymer | - | - | - | - | - | - | - | 0.5 | - | - |
| Hydroxyethyl cellulose | - | - | - | - | - | - | - | - | 0.5 | - |
| Hydroxypropyl Starch Phosphate | - | - | - | - | - | - | - | - | - | 2.0 |
| pH adjust to pH 9.0 | qs | qs | qs | qs | qs | qs | qs | qs | qs | qs |
| Water | qs | qs | qs | qs | qs | qs | qs | qs | qs | qs |

**Examples of thickened aqueous solution formulations 1 - 10**

| | **Formulation** | | | | | | | | | |
|---|---|---|---|---|---|---|---|---|---|---|
| **Ingredient** | **1** | **2** | **3** | **4** | **5** | **6** | **7** | **8** | **9** | **10** |
| Sodium sulphite | 0.1 | 0.1 | 0.1 | 0.1 | 0.1 | 0.1 | 0.1 | 0.1 | 0.1 | 0.1 |
| Ascorbic Acid | 0.1 | 0.1 | 0.1 | 0.1 | 0.1 | 0.1 | 0.1 | 0.1 | 0.1 | 0.1 |
| Citric Acid | 1.0 | 1.0 | 1.0 | 1.0 | 1.0 | 1.0 | 1.0 | 1.0 | 1.0 | 1.0 |
| Ammonium Carbonate | 5.0 | 6.0 | 5.0 | - | 4.0 | 8.0 | 2.0 | - | 5.0 | 6.0 |
| Potassium Hydrogen Carbonate | - | - | 1.5 | 2.0 | - | - | 2.0 | 3.0 | - | - |
| Ammonium Acetate | - | - | - | 2.0 | - | - | - | 3.0 | - | - |
| Oleth 10 | 1.0 | 1.0 | 1.0 | 1.0 | - | - | - | - | - | - |
| Oleth 2 | 0.8 | 0.8 | 0.8 | 0.8 | - | - | - | - | - | - |
| Oleic Acid | 0.9 | 0.9 | 0.9 | 0.9 | - | - | - | - | - | - |
| Cocamide DEA | 3.0 | 3.0 | 3.0 | 3.0 | - | - | - | | - | - |
| EDTA(tetrasodium salt) | 0.1 | 0.1 | 0.1 | 0.1 | 0.1 | 0.1 | 0.1 | 0.1 | 0.1 | 0.1 |
| p-phenylene diamine | 0.8 | | 0.6 | 0.1 | 0.8 | | 0.6 | 0.1 | 0.8 | |
| p-amino phenol | | 0.3 | | 0.4 | | 0.3 | | 0.4 | | 0.3 |
| 2,5-diaminotoluene sulphate | | 0.1 | 0.2 | | | 0.1 | 0.2 | | | 0.1 |
| m-aminophenol | 0.2 | | 0.1 | | 0.2 | | 0.1 | | 0.2 | |
| Resorcinol | | 0.5 | | 0.4 | | 0.5 | | 0.4 | | 0.5 |
| napthol | 0.03 | | 0.2 | | 0.03 | | 0.2 | | 0.03 | |
| 4-amino-2-hydroxy toluene | | 0.2 | | 0.3 | | 0.2 | | 0.3 | | 0.2 |
| Phenyl methyl pyrazalone | 0.2 | - | - | - | - | 0.1 | - | - | - | - |
| 1-hydroxyethyl-4,5-diamino pyrazole sulphate | 0.3 | - | - | - | - | 0.2 | 0.2 | | - | - |
| Basic red 51 | - | 0.1 | - | - | - | - | 0.2 | - | - | - |
| Basic yellow 87 | - | 0.2 | - | - | - | - | 0.3 | - | - | - |
| Hydrogen Peroxide (35% active) | 8.6 | 8.6 | 8.6 | 13 | 17 | 17 | 17 | 10.7 | 10.7 | 10.7 |
| Polyquaternium-22 (Merquat 295) | - | - | - | - | 0.5 | - | - | | - | - |
| Polyquaternium-37 & Mineral oil (Salcare SC95) | - | - | - | - | 0.1 | - | 0.5 | - | - | - |
| Amodimethicone (Belsil ADM1100) | - | - | - | - | - | 1.0 | - | - | - | - |
| Acrylates Copolymer (Aculyn® 33A) | 2.4 | 2.4 | 2.4 | 2.4 | - | - | - | - | - | - |
| Acrylates Steareth-20 Methacrylate Copolymer (Aculyn ® 22) | 0.5 | 0.5 | - | 1.0 | - | - | - | - | - | - |
| Xanthan gum | - | - | - | - | - | 1.0 | - | - | - | - |
| Succinoglycan | - | - | - | - | 0.8 | - | - | - | - | - |
| Carbomer | - | - | - | - | - | - | 2.0 | - | - | - |
| Acrylates/C10-30 Alkyl Acrylate Crosspolymer | - | - | - | - | - | - | - | 2.0 | - | - |
| Hydroxyethyl cellulose | - | - | - | - | - | - | - | - | 2.0 | - |
| Hydroxypropyl Starch Phosphate | - | - | - | - | - | - | - | - | - 2.0 | 2.0 |
| Propylene Glycol | 8.2 | 8.2 | 8.2 | 8.2 | 8.0 | 8.0 | 8.0 | 8.0 | 8.0 | 8.0 |
| Ethoxy Diglycol | 4.2 | 4.2 | 4.2 | 4.2 | - | - | - | - | - | - |
| pH adjust to pH 9.0 | qs | qs | qs | qs | qs | qs | qs | qs | qs | qs |
| Water | qs | qs | qs | qs | qs | qs | qs | qs | qs | qs |

## Claims

1. A hair colouring composition comprising
i) at least one source of hydrogen peroxide
ii) at least one source of carbonate, carbamate, and/or hydrogen carbonate ions
iii) at least one source of alkalising agent, wherein the source of alkalising agent is a source of ammonium ions
iv) at least one oxidative dye precursor and/or at least one preformed dye wherein said composition comprises at least greater than 4% by weight of said source of hydrogen peroxide or at least greater than 4% by weight of said source of carbonate, carbamate, and/or hydrogen carbonate ions
and wherein said composition has a pH of from 7.5 to 9.5 and wherein said composition is free of a source of radical scavengers.

2. A hair colouring composition according to claim 1, wherein said composition comprises
a. from 0.1 % to 10% by weight, preferably from 1% to 7% of hydrogen peroxide
b. from 0.1 to 10%, preferably from 0.5 to 5% by weight of said alkalizing agent
c. from 0.1 to 15%, preferably from 1% to 10% by weight of said at least one source of carbonate, carbamate and/or hydrogen carbonate ions.

3. A hair colouring composition according to claim 2, wherein said composition comprises
a. from 2% to 9% of hydrogen peroxide
b. from 0.1 to 10%, preferably from 0.5 to 5% by weight of said alkalizing agent
c. from 4 to 15% by weight of said at least one source of carbonate, carbamate and/or hydrogen carbonate ions.

4. A hair colouring composition according to claim 2, wherein said composition comprises
a. from 4% to 9% of hydrogen peroxide
b. from 0.1 to 10%, preferably from 0.5 to 5% by weight of said alkalizing agent
c. from 2% to 15% by weight of said at least one source of carbonate, carbamate and/or hydrogen carbonate ions.

5. A hair coloring composition according to claim 1, wherein said composition comprises at least one oxidative dye selected from m-aminophenol, 4-amino-2-hydroxytoluene, resorcinol, 2-methylresorcinol, 1-napthol, 2-amino-3-hydroxypyridine, m-phenylenediamine and mixtures thereof.

6. A hair colouring kit comprising
i) an individually packaged oxidizing component comprising at least one source of hydrogen peroxide
ii) an individually packaged colouring component comprising
a) at least one source of carbonate ions, carbamate ions and/or hydrogen carbonate ions, and
b) at least one alkalising agent, wherein the alkalising agent is ammonium ions
c) at least one oxidative dye precursor and/or at least one preformed dye
wherein said kit comprises at least 4% by weight of said at least one source of hydrogen peroxide or at least one source of carbonate ions, carbamate ions and/or hydrogen carbonate ions, wherein said kit is free of a source of a radical scavenger wherein the pH of the resultant mixture of the oxidizing component and the colouring component has a pH of from 7.5 to 9.5.

7. A method of oxidative colouring of hair comprising the steps of applying a composition comprising at least one source of hydrogen peroxide, at least one source of carbonate, carbamate, and/or hydrogen carbonate ions and at least one source of alkalising agent, wherein the source of alkalising agent is a source of ammonium ions, at least one oxidative dye precursor and/or at least one preformed dye, wherein said composition comprises at least 4% by weight of said source of hydrogen peroxide or at least 4% by weight of said source of carbonate carbamate, and/or hydrogen carbonate ions and wherein said composition has a pH of from 7.5 to 9.5 for at least 50% of the time period wherein said composition is applied and retained on the hair.

8. A method of oxidative colouring of hair comprising the steps of applying a composition according to any of claims 1 to 5, leaving said composition on the hair for from 2 to 60 minutes and subsequently rinsing said composition from the hair.

9. A method of oxidative colouring of hair according to claim 8, wherein said composition is retained on the hair for a time period of less than 20 minutes.

10. A method of sequential oxidative hair colouring comprising the steps of at least two sequential oxidative hair colour treatments wherein the time period between each treatment is from 1 day to 60 days, and wherein each treatment comprises the steps of providing a composition according to any of claims 1 to 5, applying said composition to the hair and retaining said composition on the hair for a time period of less than 20 minutes and subsequently rinsing said composition from the hair.

## Patentansprüche

1. Haarfärbezusammensetzung, umfassend
i) mindestens eine Wasserstoffperoxidquelle;
ii) mindestens eine Quelle von Carbonat-, Carbamat-, und/oder Wasserstoffcarbonationen;
iii) mindestens eine Quelle eines alkalisierenden Mittels, wobei die Quelle eines alkalisierenden Mittels eine Quelle von Ammoniumionen ist;
iv) mindestens einen oxidativen Farbstoffvorläufer und/oder mindestens einen vorgeformten Farbstoff;
wobei die Zusammensetzung mindestens mehr als 4 Gew.-% der Wasserstoffperoxidquelle oder mindestens mehr als 4 Gew.-% der Quelle von Carbonat-, Carbamat-, und/oder Wasserstoffcarbonationen umfasst
und wobei die Zusammensetzung einen pH-Wert von 7,5 bis 9,5 aufweist und wobei die Zusammensetzung frei von einer Quelle von Radikalfängern ist.

2. Haarfärbezusammensetzung nach Anspruch 1, wobei die Zusammensetzung Folgendes umfasst:
a. von 0,1 Gew.-% bis 10 Gew.-%, bevorzugt von 1 Gew.-% bis 7 Gew.-%, Wasserstoffperoxid;
b. von 0,1 bis 10 Gew.-%, bevorzugt von 0,5 bis 5 Gew.-% des alkalisierenden Mittels;
c. von 0,1 bis 15 Gew.-%, bevorzugt von 1 Gew.-% bis 10 Gew.-% der mindestens einen Quelle von Carbonat-, Carbamat- und/oder Wasserstoffcarbonationen.

3. Haarfärbezusammensetzung nach Anspruch 2, wobei die Zusammensetzung Folgendes umfasst:
a. von 2 % bis 9 % Wasserstoffperoxid;
b. von 0,1 bis 10 Gew.-%, bevorzugt von 0,5 bis 5 Gew.-% des alkalisierenden Mittels;
c. von 4 bis 15 Gew.-% der mindestens einen Quelle von Carbonat-, Carbamat- und/oder Wasserstoffcarbonationen.

4. Haarfärbezusammensetzung nach Anspruch 2, wobei die Zusammensetzung Folgendes umfasst:
a. von 4 % bis 9 % Wasserstoffperoxid;
b. von 0,1 bis 10 Gew.-%, bevorzugt von 0,5 bis 5 Gew.-% des alkalisierenden Mittels;
c. 0,1 bis 15 Gew.-% der mindestens einen Quelle von Carbonat-, Carbamat- und/oder Wasserstoffcarbonationen.

5. Haarfärbezusammensetzung nach Anspruch 1, wobei die Zusammensetzung mindestens einen oxidativen Farbstoff umfasst, ausgewählt aus m-Aminophenol, 4-Amino-2-hydroxytoluol, Resorcinol, 2-Methylresorcinol, 1-Napthol, 2-Amino-3-hydroxypyridin, m-Phenylendiamin und Mischungen davon.

6. Haarfärbesatz, umfassend
i) einen einzeln verpackten ersten Oxidierungsbestandteil, umfassend mindestens eine Wasserstoffperoxidquelle;
ii) einen individuell verpackten Färbebestandteil, der Folgendes umfasst:
a) mindestens eine Quelle von Carbonationen, Carbamationen, und/oder Wasserstoffcarbonationen, und
b) mindestens ein alkalisierendes Mittel, wobei das alkalisierende Mittel Ammoniumionen sind,
c) mindestens einen oxidativen Farbvorläufer und/oder mindestens einen vorgeformten Farbstoff,
wobei der Satz mindestens 4 Gew.-% der mindestens einen Quelle von Wasserstoffperoxid oder mindestens eine Quelle der Carbonationen, Carbamationen und/ oder Wasserstoffcarbonationen umfasst, wobei der Satz frei von einer Quelle eines Radikalfängers ist;
wobei der pH-Wert der resultierenden Mischung des oxidierenden Bestandteils und des färbenden Bestandteils einen pH-Wert von 7,5 bis 9,5 aufweist.

7. Verfahren der oxidativen Haarfärbung, umfassend die Schritte des Auftragens einer Zusammensetzung, die mindestens eine Wasserstoffperoxidquelle, mindestens eine Quelle von Carbonat-, Carbamat- und/oder Wasserstoffcarbonationen und mindestens eine Quelle eines alkalisierenden Mittels umfasst, wobei die Quelle des alkalisierenden Mittels eine Quelle von Ammoniumionen, mindestens ein oxidativer Farbvorläufer und/oder mindestens ein vorgeformter Farbstoff ist, wobei die Zusammensetzung mindestens 4 Gew.-% der Quelle an Wasserstoffperoxid oder mindestens 4 Gew.-% der Quelle an Carbonat-, Carbamat-, und/oder Wasserstoffcarbonationen umfasst, und wobei die Zusammensetzung einen pH-Wert von 7,5 bis 9,5 aufweist, für wenigstens 50 % des Zeitraumes, in welchem die Zusammensetzung auf das Haar aufgetragen und auf diesem belassen wird.

8. Verfahren einer oxidativen Haarfärbung, umfassend die Schritte des Auftragens der Zusammensetzung nach einem der Ansprüche 1 bis 5, des Belassens der Zusammensetzung auf dem Haar für 2 bis 60 Minuten und anschließend des Spülens der Zusammensetzung aus dem Haar.

9. Verfahren der oxidativen Haarfärbung nach Anspruch 8, wobei die Zusammensetzung für einen Zeitraum von weniger als 20 Minuten auf dem Haar belassen wird.

10. Verfahren der sequenziellen oxidativen Haarfärbung, umfassend die Schritte mindestens zweier sequenzieller oxidativer Haarfärbebehandlungen, wobei der Zeitraum zwischen den einzelnen Behandlungen von 1 Tag bis 60 Tagen beträgt, und wobei jede Behandlung die Schritte Bereitstellen einer Zusammensetzung nach einem der vorstehenden Ansprüche 1 bis 5, Auftragen der Zusammensetzung auf das Haar und Belassen der Zusammensetzung auf dem Haar für einen Zeitraum von weniger als 20 Minuten und anschließend Spülen der Zusammensetzung aus dem Haar umfasst.

## Revendications

1. Composition de coloration capillaire comprenant
i) au moins une source de peroxyde d'hydrogène
ii) au moins une source d'ions carbonate, carbamate et/ou bicarbonate
iii) au moins une source d'agent alcalinisant, dans laquelle la source d'agent alcalinisant est une source d'ions ammonium
iv) au moins un précurseur de teinture oxydative et/ou au moins une teinture préformée
où ladite composition comprend au moins plus de 4 % en poids de ladite source de peroxyde d'hydrogène ou au moins plus de 4 % en poids de ladite source d'ions carbonate, carbamate et/ou bicarbonate
et où ladite composition a un pH allant de 7,5 à 9,5 et où ladite composition est exempte d'une source d'agents anti-radicaux libres.

2. Composition de coloration capillaire selon la revendication 1, où ladite composition comprend
a. de 0,1 % à 10 % en poids, de préférence de 1 % à 7 % de peroxyde d'hydrogène
b. de 0,1 à 10 %, de préférence de 0,5 à 5 % en poids dudit agent alcalifiant
c. de 0,1 à 15 %, de préférence de 1 % à 10 % en poids de ladite au moins une source d'ions carbonate, carbamate et/ou bicarbonate.

3. Composition de coloration capillaire selon la revendication 2, où ladite composition comprend
a. de 2 % à 9 % de peroxyde d'hydrogène
b. de 0,1 à 10 %, de préférence de 0,5 à 5 % en poids dudit agent alcalifiant
c. de 4 à 15 % en poids de ladite au moins une source d'ions carbonate, carbamate et/ou bicarbonate.

4. Composition de coloration capillaire selon la revendication 2, où ladite composition comprend
a. de 4 % à 9 % de peroxyde d'hydrogène
b. de 0,1 à 10 %, de préférence de 0,5 à 5 % en poids dudit agent alcalifiant
c. de 2 % à 15 % en poids de ladite au moins une source d'ions carbonate, carbamate et/ou bicarbonate.

5. Composition de coloration capillaire selon la revendication 1, où ladite composition comprend au moins une teinture oxydative choisie parmi le m-aminophénol, le 4-amino-2-hydroxytoluène, le résorcinol, le 2-méthylrésorcinol, le 1-naphtol, la 2-amino-3-hydroxypyridine, la m-phénylènediamine et leurs mélanges.

6. Trousse de coloration capillaire comprenant
i) un composant oxydant conditionné individuellement comprenant au moins une source de peroxyde d'hydrogène
ii) un composant de coloration conditionné individuellement comprenant
a) au moins une source d'ions carbonate, d'ions carbamate et/ou d'ions bicarbonate, et
b) au moins un agent alcalinisant, dans laquelle l'agent alcalinisant est des ions ammonium
c) au moins un précurseur de teinture oxydative et/ou au moins une teinture préformée
où ladite trousse comprend au moins 4 % en poids de ladite au moins une source de peroxyde d'hydrogène ou au moins une source d'ions carbonate, d'ions carbamate et/ou d'ions bicarbonate, où ladite trousse est dépourvue de source d'un agent anti-radicaux libres.
dans laquelle le pH du mélange résultant du composant oxydant et du composant colorant a un pH allant de 7,5 à 9,5.

7. Procédé de coloration oxydative des cheveux comprenant les étapes consistant à appliquer une composition comprenant au moins une source de peroxyde d'hydrogène, au moins une source d'ions carbonate, carbamate et/ou bicarbonate et au moins une source d'agent alcalinisant, dans lequel la source d'agent alcalinisant est une source d'ions ammonium, au moins un précurseur de teinture oxydative et/ou au moins une teinture préformée, dans lequel ladite composition comprend au moins 4 % en poids de ladite source de peroxyde d'hydrogène ou au moins 4 % en poids de ladite source d'ions carbonate carbamate et/ou bicarbonate et dans lequel ladite composition a un pH allant de 7,5 à 9,5 pendant au moins 50 % de la période de temps où ladite composition est appliquée et retenue sur les cheveux.

8. Procédé de coloration oxydative des cheveux comprenant les étapes consistant à appliquer une composition selon l'une quelconque des revendications 1 à 5, à laisser ladite composition sur les cheveux pendant 2 à 60 minutes et à rincer ensuite ladite composition des cheveux.

9. Procédé de coloration oxydative des cheveux selon la revendication 8, dans lequel ladite composition reste sur les cheveux pendant une période de temps de moins de 20 minutes.

10. Procédé de coloration capillaire oxydative séquentielle comprenant les étapes d'au moins deux traitements oxydatifs séquentiels de coloration capillaire, dans lequel la période de temps entre chaque traitement va de 1 jour à 60 jours, et dans lequel chaque traitement comprend les étapes consistant à fournir une composition selon l'une quelconque des revendications 1 à 5, à appliquer ladite composition sur les cheveux et à faire rester ladite composition sur les cheveux pendant une période de temps de moins de 20 minutes et à rincer ensuite ladite composition des cheveux.
